Europäisches Patentamt

European Patent Office

Office européen des brevets

(19)

(11) Publication number: **0 289 616**
**A1**

(12)
# EUROPEAN PATENT APPLICATION
published in accordance with Art. 158(3) EPC

(21) Application number: **87906789.0**

(22) Date of filing: **19.10.87**

Data of the international application taken as a basis:

(86) International application number:
**PCT/JP 87/00800**

(87) International publication number:
**WO 88/03163 (05.05.88 88/10)**

(51) Int. Cl.⁴: **C 12 N 5/00**, C 12 N 5/02

(30) Priority: **21.10.86 JP 250014/86**

(43) Date of publication of application: **09.11.88**
**Bulletin 88/45**

(84) Designated Contracting States: **DE FR GB**

(71) Applicant: **KYOWA HAKKO KOGYO KABUSHIKI KAISHA, 6-1, Ohte-machi 1-chome, Chiyoda-ku Tokyo 100 (JP)**

(72) Inventor: **HOSOI, Shinji, Popuragaoka Coopu 16-104, 2-21-2, Naruse, Machida-shi Tokyo 194 (JP)**
Inventor: **MIOH, Hiroyuki, 2 Greenwood Street, Lake Placid, NY 12946 (US)**
Inventor: **SATO, Seiji, 1511-7, Okada Atsugi-shi, Kanagawa 243 (JP)**
Inventor: **FUJIYOSHI, Nobuo, 1-12-2, Asahi-machi Machida-shi, Tokyo 194 (JP)**

(74) Representative: **Lambert, Hugh Richmond et al, D. YOUNG & CO. 10 Staple Inn, London, WC1V 7RD (GB)**

(54) **PROCESS FOR MULTIPLICATION OF ANIMAL CELLS.**

(57) Improved process for multiplication on animal cells, which comprises culturing animal cells in a medium containing blood serum or a cell multiplication factor in one section separated from another section by a partition wall, and conducting perfusion culturing in a medium not containing serum nor cell multiplication factor in the another section. Substances useful as medicines can by yielded in large amounts by multiplication of the obtained animal cells.

00289616

SPECIFICATION

METHOD FOR PROPAGATION OF ANIMAL CELLS

Field of the Invention

The present invention relates to a method for propagation of animal cells.

By propagating animal cells, substances (for example, interferon) useful for pharmaceutical preparations, etc. can be produced in large quantities.

Prior Art

Upon cultivation of animal cells, it has been hitherto required to add sera, cell growth factors, etc. to media due to nature of cells. However, since these components are expensive or in order to remove uneven cell propagation due to difference in lots of these components, development on serum-free media is now under way.

Currently, however, it is impossible to cultivate all of animal cells (for example, normal lymphocytes, normal bone marrow cells, etc.) in serum-free media. On the other hand, as the method for efficiently cultivating animal cells using media containing serum, cell growth factor, etc., development has been made on the high density cultivation method and various types of perfusion cultivation method are known [Science, 164, 555 (1969), Develop. Biol. Standard, 55, 31 (1982)]. According to the high density cultivation method, perfusion cultivation is performed using media containing serum or cell growth factor. Accordingly, in order to replenish fresh media, a large quantity of serum or cell growth factor are required.

Development on a method for propagating animal cells in large quantities by adding a small quantity of serum or cell growth factor to a medium has been desired.

- 2 -

00289616

## Disclosure of the Invention

According to the method of the present invention, in the perfusion cultivation method using animal cells, animal cells are subjected to perfusion cultivation in a medium containing serum or cell growth factor which is put in one compartment, separated by a partition wall with the other compartment full of serum- or cell growth factor-free medium to thereby propagate animal cells efficiently in large quantities using a small quantity of serum or cell growth factor.

As animal cells to be used in the present invention, mention may be made of lymphocytes, lymphoblasts, hybridomas, epithelial cells, fibroblasts, bone marrow cells, etc. Specific examples include CTLL-2 (ATCC TIB214) derived from murine T cells (lymphocytes) and Namalva cells (ATCC CRL 1432) of human lymphoblasts, and normal human lymphocytes.

Media to be used in the present invention are as follows.

As the media directly contacting and cultivating animal cells, media composed of serum or cell growth factor and basal media (media containing amino acids, vitamins, inorganic salts, sugars, nucleic acid-associated substances, etc.) can be used. As the dialyzing media without directly contacting animal cells, basal media are used.

As sera, mention may be made of sera collected from human, bovine, horse, etc.

Examples of the cell growth factor include proteinaceous or peptide-like cell growth factor, for example, an epithelial cell growth factor, a fibroblast growth factor, interleukin 2 (hereinafter referred to as IL-2), platelet-derived growth factor, transforming growth factor $\alpha$, somatomedin, etc.

As the amino acids contained in basal media, arginine, cystein, glutamine, histidine, isoleucine, leucine, lysine, methionine, phenylalanine, threonine,tryptophan, tyrosine, valine, etc. may be mentioned.

As the vitamins, mention may be made of vitamin A, thiamine, riboflavin, nicotinamide, pantothenic acid, pyridoxal, biotin, choline, etc.

As the inorganic salts, mention may be made of sodium chloride, potassium chloride, calcium chloride, magnesium chloride, sodium dihydrogenphosphate, calcium dihydrogenphosphate, copper sulfate, iron sulfate, zinc sulfate, sodium hydrogencarbonate, etc.

As the sugars, mention may be made of glucose, fructose, galactose, sucrose, etc.

As the nucleic acid-associated substances, mention may be made of 5'-inosinic acid, 5'-guanylic acid, inosine, etc.

Examples of the basal media include RPMI-1640, DME, MEM, Ham F-10, Ham F-12, DM-160, etc.

In addition, antibiotics (streptomycin, penicillin, etc.), sodium bicarbonate, insulin, pyruvic acid, selenious acid, ethanolamine, surfactants, etc. may also be added, if necessary.

As the partition wall to be used in the present invention, any partition wall can be used as long as it does not allow animal cells or sera and cell growth factors which are high molecular weight substances to pass but allow low molecular weight substances such as amino acids, vitamins, inorganic salts, sugars, nucleic acid-associated substances, etc. to pass therethrough. Examples include a membrane, a porous plate, microcapsules, etc.

As specific membranes, mention may be made of synthetic resin ultrafiltration membranes having nominal cut-off level at a molecular weight of 3,000 to 100,000, preferably 6,000 to 30,000, for example, membranes of nylon 66, cellulose triacetate, mixed cellulose ester, regenerated cellulose, nitrocellulose, acrylonitrile, vinyl chloride, polypropylene, Teflon, etc.

- 4 -

00289616

As the porous plate, mention may be made of a metal sintered plate, a ceramic plate, etc.

As materials to be used in microencapsulating the media containing animal cells and serum or cell growth factor, agarose, sodium alginate, chitosan, kappa-carragheenan, etc. are used.

Cultivatation performed in case that one compartment separated by the partition wall is a culture vessel and the other is a dialysis vessel, is described below.

A concentration of animal cells in the culture vessel is in a range of $10^3$ to $10^9/m\ell$, preferably $10^4$ to $10^6/m\ell$. Cultivation in the culture vessel in performed by spinner culture at 30 to 38°C in a range of 10 to 150 rpm, preferably 30 to 50 rpm.

Cultivation in the dialysis vessel in which no aminal cell is contained is performed by spinner culture at 30 to 38°C in a range of 10 to 150 rpm, preferably 30 to 50 rpm. Replacement of media is performed at a rate which varies depending upon the cell number or the metabolic rate. As a media for replacement, the basal medium described above can be used.

Generally, replacement of the medium is linearly speeded up from the rate of the culture volume/day to the rate of 2 to 5 times the culture volume/day, correspondingly to the increased cell number after the cell number reaches $10^6/m\ell$. In addition, sugars such as glucose, etc., amino acids, etc. may also be appropriately added to the medium, and oxygen may also be passed therethrough. The serum concentration in the upper culture vessel separated by the membrane does not change even though serum or cell growth factor is not added to the medium to be replaced, but the amount of serum reduced from the medium by being adsorbed to cells or the amount of serum to be metabolized may only be supplemented. In the case of supplementing the cell growth factor, an amount corresponding to that consumed by the increased cell number is replenished.

00289616

According to this method, approximately 10 times the cell number can be obtained using the same addition amount of serum or cell growth factor as used in batch culture conventionally performed.

Next, description is made on the case where the partition wall is microcapsures.

As raw materials for encapsulation, those described above can be used.

In the case of agarose, 2.5 to 10% agarose is mixed at 40°C with $5 \times 10^5$ to $6 \times 10^6$/mℓ of cells cultures at 37°C to give 1.25 to 5% gel. Then, the gel is dropwise added to a paraffin solution. Upon cooling with agitation, the cells are enclosed within spherical agarose particles. In the case of sodium alginate, $5 \times 10^5$ to $6 \times 10^6$/mℓ of cells are mixed with sodium alginate in a final gel concentration of 1 to 4%. The mixture is dropwise added to 50 mM calcium chloride to perform microencapsulation.

In the case of kappa carragheenan, $5 \times 10^5$ to $6 \times 10^6$/mℓ of cells are mixed with kappa carragheenan in a final gel concentration of 1 to 4%. The mixture is dropwise added to 2% potassium chloride to perform microencapsulation.

The above-mentioned microcapsule particles in which the animal cells are enclosed may sometimes be used after washing with polycations such as polyethyleneimine, polypropyleneimine, polyvinylamine, polylysine, etc. for purposes of improving permeability.

The microcapsule particles are aseptically filtered and washed with the basal medium. Then, perfusion cultivation is performed using the basal medium, whereby cells can be propagated in the gel in a high concentration.

Cultivation is performed in a manner similar to above, by spinner culture at 30 to 38°C in a range of 10 to 150 rpm, preferably 30 to 70 rpm. Replenishment of the medium is performed at a rate suited to cell number and metabolic rate.

Brief Description of the Drawing

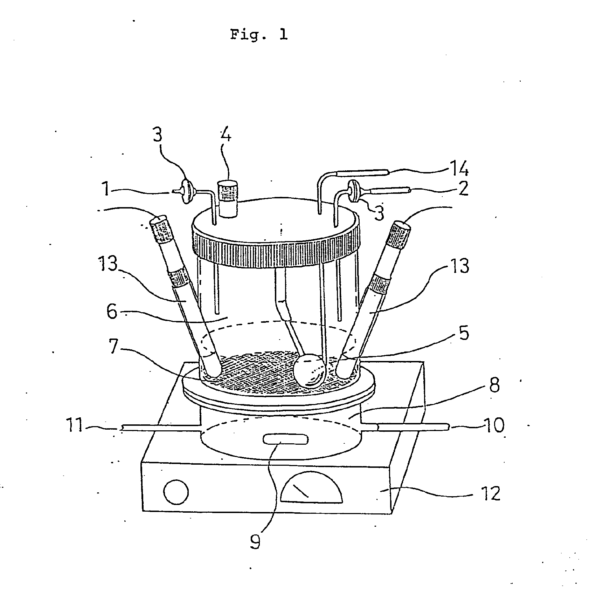

Figure 1 shows an example of a device for dialytic perfusion cultivation comprising a culture vessel and a dilysis vessel which is used for the perfusion cultivation method of the present invention.

1 ··· aeration line (inlet)
2 ··· aeration line (outlet)
3 ··· sterile filter
4 ··· opening for inoculation
5 ··· stirring ball
6 ··· culture vessel
7 ··· membrane
8 ··· dialysis vessel
9 ··· stirring bar
10 ··· inlet for medium to be dialyzed
11 ··· outlet for medium to be dialyzed
12 ··· magnetic stirrer
13 ··· sensor
14 ··· sampling line

Best Mode for Practice of the Invention

Example 1

Using a device for dialysis perfusion cultivation illustrated in Figure 1, dialysis perfusion cultivation of murine T cell-derived CTLL-2 (ATCC TIB214) was carried out. A the device for dialysis perfusion cultivation, one composed of two vessels is used: one being a culture vessel at the upper compartment and the other being a dialysis vessel at the lower compartment, separate by a dialysis membrane (Spectra/pore 2, Yeda Trading Co.) having nominal cut-off level of a molecular weight of 12,000 to 14,000. At that time, 200 ml of the cultivation amount was contained in 500 ml-cultivation vessel, and 300 ml of a medium was contained in a dialysis vessel.

RPMI-1640 medium (manufactured by Nissui Pharamaceutical Co., Ltd.) was supplemented with 10% v/v bovine fetal serum, 4 mM glutamine, 25 µg/mℓ streptomycin, 25 U/mℓ penicillin, 10 mM Hepes buffer and 0.19% w/v sodium bicarbonate, and 1250 U/mℓ IL-2 was added thereto to make the culture solution. As the dialyzing solution, a medium obtained by removing bovine fetal serum and IL-2 from the culture solution described above and supplementing 3 µg/mℓ insulin, 5 mM pyruvic acid, $1.25 \times 10^{-7}$M selenious acid, 1 mg/mℓ galactose and 0.1% Pepol B-188 (Toho Chiba Chemical Industry Co., Ltd.) was used.

CTLL-2 was inoculated in the culture vessel in $6 \times 10^4$/mℓ. Viability of cells at this time was 93.8%.

Without making replacement of the medium in the dialysis vessel for 2 days after initiation of the cultivation, spinner culture was performed at a rotation number of 50 rpm at 37°C. Thereafter, the cultivation was continued while conducting dialysis. Dialysis was conducted at a flow rate of 500 mℓ/day on the third day, 750 mℓ/day on the fourth and fifth day and subsequently 1 liter/day.

Aeration was performed with 5% $CO_2$ for 3 days, 1% $CO_2$ on the fourth day because of reduced pH, and then 0.5% $CO_2$ on the fifth day and thereafter. CTLL-2 was propagated to $1 \times 10^7$/mℓ in 5 days.

Example 2

Human lymphoblasts-derived Namalva cells (ATCC CRL 1432) were cultivated at 37°C and 50 rpm for 2 days in a medium obtained by supplementing 10% bovine fetal serum, 4 mM glutamine, 25 µg/mℓ streptomycin, 25 µg/mℓ penicillin, 10 mM Hepes buffer and 0.16% w/v sodium bicarbonate to RPMI-1640 medium (manufactured by Nissui Pharmaceutical Co., Ltd.).

The cultivated cells described above were suspended in the aforesaid medium containing 2% sodium alginate in $6.0 \times 10^5$/mℓ. The suspension was charged in a syringe and dropwise added to physiological saline containing 2% calcium chloride. By this operation, the cells were enclosed within spherical capsules (microcapsules) of sodium alginate having a diameter of 600 μm. At this time, a living cell density in the capsules was $5.1 \times 10^5$/ml. Serum was simultaneously enclosed in the capsules.

In a 500 ml-high density culture device (manufactured by Shibata Hario Co., Ltd.) of an inverted conical shape was put 400 ml of serum-free medium (one obtained by removing serum from the medium described above), and microencapsulated cells were washed with 40 ml of 0.1% w/v poly-L-lysine (manufactured by Sigma Inc.) calculated as the inner content of the capsules and inoculated into the medium. One day after the inoculation, perfusion was carried out with the serum free medium at a rate of 400 ml/day. Four days after, the flow rate was 800 ml/day. When cultivation was performed for 10 days, the cell number after cultivation reached $2.1 \times 10^7$/ml. The cultivation was performed at 37°C at a spinner rate of 50 rpm in an aeration amount of 2 l/hr.

Example 3

Normal human lymphocytes were cultivated using the device for dialysis perfusion cultivation illustrated in Figure 1. As the cultivation medium, RPMI-1640 medium supplemented with 10% human plasma, 4 mM glutamine, 10 μg/ml gentamicin, 0.2% sodium bicarbonate and 2 U/ml of IL-2 (manufactured by Takeda Chemical Industries Co., Ltd.) was used. As the dialyzing medium, a medium obtained by removing the plasma and IL-2 from the cultivation medium described above was used.

Peripheral blood cells, $1 \times 10^7$/ml, collected from healthy donor were suspended in the culture medium described above. While supplying 5% carbon dioxide to adjust pH to 6.8 to 7.2, cultivation was carried out at agitation of a rotation number of 50 rpm at 37°C. Replacement of the medium in the dialysis vessel started from 500 ml/day, which gradually increased up to 2,500 ml/day, and the cultivation was continued for 20 days.

IL-2 was supplemented every second day to keep the concentration of 20 U/m$\ell$. As a result, 16 days after, the cells increased up to $1.97 \times 10^7/m\ell$ which could be maintained on the 30th day.

## Claims

1. A method for propagating animal cells in perfusion cultivation using animal cells which comprises cultivating the animal cells in a medium containing serum or a cell growth factor put in one compartment separated by a partition wall with the other compartment full of a serum- or cell growth factor-free medium.

2. A method as claimed in claim 1 wherein said partition wall is a membrane, a porous plate or microcapsules.

3. A method as claimed in claim 2 wherein said membrane is an ultrafiltration membrane comprising a synthetic resin having nomianl cut-off level of a molecular weight of 3,000 to 100,000.

4. A method as claimed in claim 2 wherein said porous plate is a metal sintered plate or a ceramic plate.

5. A method as claimed in claim 2 wherein said microcapsules comprise sodium alginate, agarose or kappa carragheenan.

6. A method as claimed in claim 1 wherein said cell growth factor is a proteinaceous or peptide-like cell growth factor.

7. A method as claimed in claim 6 wherein said proteinaceous or peptide-like cell growth factor is an epithelial cell growth factor, a fibroblast growth factor, interleukin 2, a platelet-derived growth factor, a transforming growth factor $\alpha$, or somatomedin.

8. A method as claimed in claim 1 wherein said animal cells are lymphocytes, lymphoblasts, hybridomas, epithelial cells, fibroblasts or bone marrow cells.

Fig. 1

00289616

# INTERNATIONAL SEARCH REPORT

International Application No PCT/JP87/00800

## I. CLASSIFICATION OF SUBJECT MATTER (if several classification symbols apply, indicate all) 3

According to International Patent Classification (IPC) or to both National Classification and IPC

Int.Cl⁴  C12N5/00, C12N5/02

## II. FIELDS SEARCHED

### Minimum Documentation Searched 4

| Classification System | Classification Symbols |
|---|---|
| IPC | C12N5/00, C12N5/02 |

Documentation Searched other than Minimum Documentation
to the Extent that such Documents are Included in the Fields Searched 5

## III. DOCUMENTS CONSIDERED TO BE RELEVANT 14

| Category * | Citation of Document, 16 with indication, where appropriate, of the relevant passages 17 | Relevant to Claim No. 18 |
|---|---|---|
| A | Hagiwara Bunji "Maku niyoru Bunri-ho" 10 October 1982 (10. 10. 82) Kodansha, Ltd. P.256-261 | 1-8 |

* Special categories of cited documents: 15

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search 2 | Date of Mailing of this International Search Report 2 |
|---|---|
| January 6, 1988 (06.01.88) | January 11, 1988 (11.01.88) |

| International Searching Authority 1 | Signature of Authorized Officer 20 |
|---|---|
| Japanese Patent Office | |